# EUROPEAN PATENT SPECIFICATION

(11) **EP 1 794 316 B1**
(45) Date of publication and mention of the grant of the patent: **03.10.2018**
(21) Application number: 05793508.2
(22) Date of filing: 30.08.2005
(51) Int. Cl.: G01N 33/48, G06F 19/00, A23K 50/40

(54) **GENOME-BASED DIET DESIGN**
DIÄTPLAN AUF GENOMBASIS
CONCEPTION DE RÉGIME ALIMENTAIRE BASE SUR LE GÉNOME

(30) Priority: 30.08.2004 US 605573 P
(43) Date of publication of application: 13.06.2007
(73) Proprietor: Hill's Pet Nutrition, Inc., Topeka, KS 66603 (US)
(72) Inventor: FRIESEN, Kim, Gene, Carthage, Indiana 46115 (US); YAMKA, Ryan, Michael, Succasunna, NJ 07876 (US)
(74) Representative: Wichmann, Hendrik
(86) International application number: PCT/US2005/030567
(87) International publication number: WO 2006/026512

(56) References cited:
- WO-A2-02/47473
- WO-A2-2004/113570
- US-A- 6 156 355
- US-B1- 6 287 254
- US-B1- 6 287 254
- US-B1- 6 493 641
- US-B1- 6 493 641
- US-B1- 6 669 975
- US-B1- 6 669 975
- US-B2- 6 576 280
- PARKER H G ET AL: "Genetic structure of the purebred domestic dog" SCIENCE, AMERICAN ASSOCIATION FOR THE ADVANCEMENT OF SCIENCE, WASHINGTON, DC; US LNKD- DOI:10.1126/SCIENCE.1097406, vol. 304, no. 5674, 21 May 2004 (2004-05-21), pages 1160-1164, XP002297783 ISSN: 0036-8075
- PARKER ET AL.: 'Genetic Structure of the Purebred Domestic Dog' SCIENCE vol. 304, no. 5674, 21 May 2004, pages 1160 - 1164, XP002297783

## Description

### FIELD OF THE INVENTION

The present invention generally relates to methods of formulating and preparing animal foods based on an animal's genome as defined in the claims.

### BACKGROUND OF THE INVENTION

Humans continually interact with certain species of animals for purposes such as hunting, herding, security, and companionship, Among such animals are dogs and cats. Dogs, in particular, have been bred by humans to bring out characteristics that are important for specific roles that a dog plays in relation to a human. A variety of dog morphologies have existed for millennia, and selective breeding has tended to lead to a degree of reproductive isolation among breeds exhibiting these different morphologies. This reproductive isolation has been formalized since the mid 19th century by the advent of breeding in clubs according to breeding standards, which are well documented in such groups as the American Kennel Club (AKC), European Kennel Clubs and the Japanese Kennel Club. The promulgation of the "breed barrier" rule permits a dog to be registered to a recognized breed only if the dam and the sire are registered members of that breed. This rule has ensured a relatively closed genetic pool among dogs of each breed.

Animal nutritional needs have been known for centuries and, in the last hundred years, a large industry has developed to manufacture and distribute animal foods, especially canine and feline foods, to retail outlets including grocery, feed, and pet stores. The industry has distinguished food for different animal attributes or phenotypes. Such attributes or phenotypes have included animal size, age, or body condition and in some cases foods have been proposed or marketed for one or more specific breeds or phenotypically defined breed types, including AKC recognized breed groups.

Various food recipes, said to be adapted for each of seven canine breed groups, are proposed in U.S. Patent No. 6,156,355 to Shields & Bennett, Pet food formulas said to have "added special ingredients" for dogs of particular breed groups, which appear to correspond exactly to AKC breed groups, are described in a series of web pages accessible from http://www.naturesrecipe.com/pages/dogproducts/breed. Design of formulations for canines has also been proposed based on particular phenotypic differences such as growth rate in large breeds versus smaller breeds. See for example U.S. Patent No. 5,851,573 to Lepine et al. The greater growth rate in large breed canines can lead to orthopedic failure (e.g., hip dysplasia) due to an imbalance between rapid muscle growth and bone development.

It has been proposed that foods can be formulated specifically for an individual companion animal based on phenotypic characteristics of the individual animal. See, e.g., U.S. Patent No. 6,669,975 to Abene et al.

Healthy nutrition of companion animals is one of the most important aspects of pet care. Many animal owners have difficulty in determining if their animal is receiving a well-balanced and healthy diet. While people are becoming much more aware regarding their own personal nutrition, they have relatively little knowledge of their dietary requirements.

With recent innovations in health and medicine based on information from genome projects, genetics is becoming a more important component in determining health and nutrition programs. New methods for designing nutrition and health programs including formulating foods for animals based on genomic information would represent a useful advance in the art.

WO 2004/113570 provides a method for assessing a nutritional requirement, diseases susceptibility or behavioural characteristics of a dog.

US 6,493,641 discloses a method and apparatus for customising pet food.

US 6,576,280 discloses systems for customising pet food.

WO 02/47473 discloses a computer system for determining a customised animal feed.

US 6,156,355 discloses breed specific canine food formulations.

US 6,287,254 is directed to animal health diagnosis.

US 6,669,975 discloses a customised dietary health maintenance system for pets.

Parker et al. 2004 (Science, Vol. 304, No. 5674, Pages 1160 to 1164) discloses a study of genetic relationships in domestic dog breeds.

### SUMMARY OF THE INVENTION

The present invention provides a method for providing nutrition for an animal comprising (a) identifying a genome-based breed cluster to which the animal belongs; and (b) formulating a food composition for the canine having a nutritional formula matched at least in part to nutritional needs of canines in the breed cluster; and preparing a food composition for the canine; wherein the genome-based breed cluster is selected from the group consisting of Cluster I, Cluster II, Cluster III, and Cluster IV as defined in Parker et al., Science 304: 1160-1164, 2004, and wherein steps (a) and (b) are performed using a computer-aided system.

The invention further provides a computer-aided system comprising one to a plurality of user-interfaceable media for implementing the method of the invention, comprising: (a) a first data set relating to a plurality of breed clusters to genome-related attributes of each breed cluster, wherein the genome-based breed clusters are Cluster I, Cluster II, Cluster III and Cluster IV as defined in Parker et al., Science 304: 1160-1164, 2004; and (b) a first algorithm capable, while drawing on the first data set, of (i) processing input data on one or more genome-related attributes of the canine to define a breed cluster to which the canine can be allocated, and (ii) formulating a food composition for the canine having a nutritional formula matched at least in part to nutritional needs of canines in the breed cluster.

Further areas of applicability of the present invention will become apparent from the detailed description provided hereinafter.

### BRIEF DESCRIPTION OF THE DRAWING

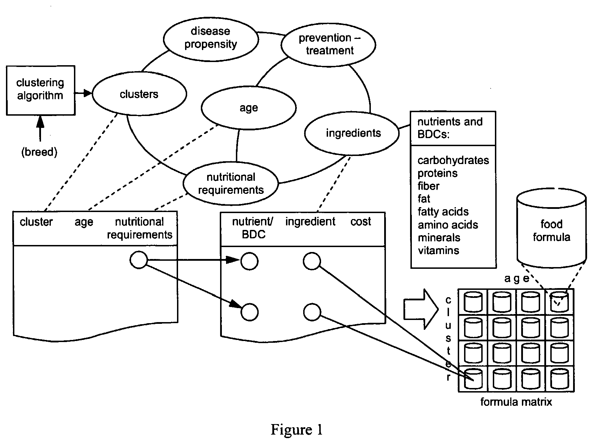

Figure 1 is a flow chart showing relationships of breed cluster, age and nutritional needs to constructing a matrix of food compositions for an animal species.

The drawing is intended to exemplify general characteristics of certain embodiments of the invention, and may not precisely reflect the characteristics of any given embodiment.

### DETAILED DESCRIPTION OF THE INVENTION

The present invention as defined in the claims provides methods of formulating foods based at least in part on an canine's genome.

The invention provides a new approach to enhancing canine nutrition and health care based on the genotype of a canine. Unlike previous efforts to provide genotype-specific foods, e.g., breed-specific foods, methods provided herein utilize a more comprehensive genomic profile of a canine coupled with rigorous statistical analysis, to define breed clusters that exhibit a nutritionally appropriate degree of genetic similarity within clusters and more marked genetic diversity between clusters. Breed clusters so defined are described herein as "genome-based" breed clusters. Without being bound by theory, it is believed that members of such a breed cluster typically have a common phylogeny, i.e., arc descended from a single ancestral population. Except where the context demands otherwise, the term "breed cluster" herein means a genome-based breed cluster, as opposed to a cluster of breeds grouped according to Criteria other than genotype. Thus traditional classifications of animal breeds based on phenotypic criteria such as AKC's classification of canine breeds into seven groups (sporting, hound, working, terrier, toy, non-sporting and herding groups) do not meet the definition of "breed clusters" as understood herein.

As defined in the claims, the present invention provides methods for formulating a canine food. In various embodiments, phenotypic information includes disease prevalence of a breed cluster. In other embodiments, phenotypic information comprises physical attributes characteristic of a breed cluster. In various embodiments, the method includes formulating the food based in part on an animal's age or age group. Such age groups include, in the case of canines, puppy, adult, senior and geriatric age groups. In various embodiments, the method also includes formulating the food to address a disease that is manifested in the animal, e.g., through prevention and/or treatment.

The term "diet" as used herein means the food or drink consumed by an animal and may include a daily ration provided by an owner. A daily ration may include any suitable food composition that provides adequate nutrition for the animal, For example, a typical canine food composition may contain from about 10% to about 30% by weight fat, about 22% to about 44% by weight protein and about 10% by weight total dietary fiber, on a dry matter basis.

However, foods selected or prepared by methods described herein are not limited to any specific ranges of ratio or percentage of these or other nutrients, A nutrient is any dietary constituent that helps support life and/or health. Table 1 provides examples of nutrients that have important roles in an animal's health.

**Table 1. Typical components of a companion animal diet**

| | |
|---|---|
| Proteins | Main element of body tissues including muscles, blood, skin, organs, hair and nails. |
| Carbohydrates | Source of energy for the body's tissues. |
| Fats | Fats absorb, store and transport vitamins, moisturize skin and coat, make healthy food taste great and supply energy. |
| Water | The most critical nutrient for survival. |
| Vitamins | Assist in maintaining an animal's metabolism. |
| Minerals | Necessary to develop healthy skin and hair, proper skeletal support and development. Minerals are usually abundant in pet food ingredients. |

Food compositions can be selected or prepared by the present methods for a canine.

A "companion animal" herein is an animal of any species kept by a human owner or caregiver as a pet, or any animal of a variety of species that have been widely domesticated as pets, including dogs (Canis familiaris) and cats (Felis domesticus), whether or not the animal is kept solely or partly for companionship. Thus, "companion animals" herein include working dogs, farm cats kept for rodent control, etc., as well as pet dogs and cats.

An "owner" herein is a person responsible for looking after an animal and most particularly for feeding the animal, and does not necessarily hold legal ownership of the animal, and can therefore be, for example, a "keeper," "caregiver" or "guardian" of the animal. An owner can be one to a plurality of persons sharing such responsibility, for example, members of a family, or a person or persons to whom such responsibility is delegated or entrusted. An end-user of a food composition prepared according to a method of the invention, is illustratively an owner of a companion animal as defined above.

will be understood to embrace clusters of animal subpopulations whether or not these have arisen by human-directed breeding. In one embodiment, a method comprises identifying a breed cluster to which the animal belongs, establishing a set of nutritional needs for wellness characteristic of that breed cluster, and selecting a food for the animal having a nutritional formula that is matched at least in part to those nutritional needs. The food can be selected from a list of preset options or, alternatively, customized to the animal,

Such a method can further comprise preparing the food by compounding ingredients that provide bioactive dietary components (BDCs) in amounts and ratios consistent with a nutritional formula matched at least in part to the identified nutritional needs. This food preparation step can occur before, simultaneously with or after the food selecting step. For example, a range of foods can be pre-manufactured for a plurality of breed clusters; and upon identification of a breed cluster to which a particular animal belongs (i.e., allocation of the animal to a breed cluster), one or more of the pre-manufactured foods can be selected accordingly. Alternatively, a food defined by its nutritional formula can be selected (based at least in part on the breed cluster identified for the animal), and subsequently prepared according to that nutritional formula.

"Wellness" of an animal herein encompasses all aspects of the physical, mental, and social well-being of the animal, and is not restricted to the absence of infirmity. Wellness attributes include without limitation states of disease or physiological disorder, states of parasitic infestation, hair and skin condition, sensory acuteness, dispositional and behavioral attributes, and cognitive function. Nutritional needs for wellness thus can be satisfied not merely by supplying sufficient of the basic nutrients required for maintenance of life, but by supplying amounts and balances of different nutrients and BDCs that, when fed to the animal, promote one or more aspects of wellness.

A "bioactive dietary component" or "BDC" as used herein is a material that, when included in an animal's diet at an appropriate level, promotes wellness of the animal. BDCs include materials typically thought of as nutrients as well as materials that are not necessarily essential for life. BDCs include chemical entities, most of which occur naturally in certain foods, but can in many cases be prepared by microbiological (e.g., fermentation) or synthetic processes. Certain biological materials, especially botanicals, can also be considered BDCs. In many of these, a bioactive chemical entity has been identified; even where a bioactive component is known, other, unknown, bioactive components may be present and contribute to the bioactive effect of the biological material.

Nutritional promotion of wellness can include enhancing an aspect of health of the animal, e.g. by preventing, attenuating or eliminating at least one disease state in the animal. Such a disease state can be one to which the breed cluster is predisposed, and can be, but is not necessarily, present in the animal. Such a disease state can be asymptomatic. A cluster of two or more disease states can be simultaneously prevented, attenuated or eliminated. Nutritional promotion of wellness by a method of the invention can be accompanied by medical intervention; for example, the food selected according to a method of the invention can be adapted for use in conjunction with medication to prevent, attenuate or eliminate at least one disease state.

Nutritional promotion of wellness can include reducing or eliminating a dispositional or behavioral problem. Nutritional promotion of wellness further encompasses improved nutritional management of an animal at specific stressful stages in its life, even when no disease or disorder is present, e.g., during growth and development of a kitten or puppy; during gestation and lactation; before and after surgery, e.g., spaying; and before, during, and after long-distance transportation. Nutritional promotion of wellness further encompasses enhancing any aspect of health in offspring of the animal, e.g., by in utero nutrition when feeding a gestating female animal.

Conditions adverse to wellness encompass not only existing diseases and physiological (including mental, behavioral, and dispositional) disorders, but predisposition or vulnerability to such diseases or disorders. Asymptomatic as well as outwardly evident diseases and disorders are likewise encompassed.

Promotion of wellness of an animal is to be understood herein as further encompassing reducing nuisance to humans living in proximity to the animal. Examples of such nuisance include, without limitation, excessive shedding, odor of excreta (including feces, intestinal gas and urine), and allergenicity.

The nutritional needs for wellness of animals of a particular breed cluster can be based at least in part on one or more phenotypic attributes characteristic of the breed cluster. Such phenotypic attributes can include physical attributes, such as size, coat type or activity level, cognitive attributes such as trainability, and/or prevalence of or predisposition to one or more diseases, e.g., cardiovascular diseases, obesity, diabetes, dermatitis, eye diseases, kidney diseases, thyroid diseases, arthritis or age-related degenerative diseases. Phenotypic attributes characteristic of a breed cluster can be derived at least in part from data, published or otherwise, on phenotypic attributes of individual breeds within the breed cluster.

Breeds of many animals, including canine breeds, have traditionally been grouped on the basis of their roles in human activities, physical phenotypes, and historical records. Dogs represent a particularly large diversity of phenotypic characteristics. The term "phenotype" as used herein means one or more observable functional or structural characteristics of an organism as determined by interaction of the genotype of the organism with the environment in which it exists. The term "genotype" means the genetic constitution of an organism with respect to one or more observable characteristics, and corresponds to the alleles present at one or more specific loci. The genotype comprises genetic information carried on chromosomes and extrachromosomally. The term "genome" generally means all the genetic material of an organism, but as used herein the term "genome" refers to the total genetic constitution or any fraction thereof sufficiently large to be amenable to analysis for the purpose of determining degree of genetic similarity or difference between organisms or populations of organisms.

Methods of the present invention can utilize classification of breeds, e.g., canine breeds, based on genetic variation independently of other factors, to design not only food formulations but complete health and nutrition programs for clusters of breeds that are established based on genetic similarities among breeds within a cluster. In some embodiments, this classification supports a subset of traditional breed groupings. In some embodiments, classification based on genetic variation reveals previously unrecognized relationships among breeds. In various embodiments of the present invention, an accurate understanding of genetic relationships among breeds lays the foundation for a complex genetic basis for morphology, behavior, activity, body composition, aging, and disease susceptibility.

Currently more than 400 breeds of dogs are described in the world today, with about 152 of these breeds recognized by AKC. Within purebred breeds over 350 genetic disorders are described, and many of these are restricted to specific breed, breed type, or genetic disposition. Patterson et al., J. Am. Vet. Med. Assoc. 193(9):1131-1144 (1998). Many of these mimic common human disorders and their restriction to particular breeds or groups of breeds is believed to be a result of aggressive breeding programs used to generate specific morphologies. Animals of mixed breed can be assigned to a breed cluster for purposes of the present invention, e.g., through knowledge of their parentage or breed heritage,

Phylogenetic analysis of an animal species or population is known. An example of such an analysis is found in Parker et al., Science 304:1160-1164 (21 May 2004).

This example used molecular markers of 85 domestic dog breeds to study genetic relationships. Differences among the breeds accounted for about 30% of genetic variation. Microsatellite typing of the 85 breeds was combined with phylogenetic analysis and modern genetic clustering allowing for a definition of related groups of breeds. In this example, to assess the amount of sequence variation in purebred dogs, 19,867 base pairs of noncontiguous genomic sequence in 120 dogs representing 60 breeds were resequenced. Further, 75 SNPs were identified with minor allele frequencies ranging from about 0.4% to about 48%, fourteen of the SNPs being breed specific. When all dogs were considered as a single population, the observed nucleotide heterozygosity was 8 x 10⁻⁴, essentially the same as that found for the human population.

To further characterize genetic variation within and among breeds, 96 microsatellite loci in 414 purebred dogs representing 85 breeds were genotyped. It was predicted that, because of the existence of breed barriers, dogs from the same breed would be more similar genetically than dogs from different breeds. To test this prediction, the proportion of genetic variation among individual dogs that could be attributed to breed membership was estimated. An analysis of molecular variance in the microsatellite data showed that variation among breeds accounts for more than 27% of total genetic variation, Similarly, the average genetic distance between breeds calculated from the SNP data is F_{ST} = 0.33. These observations are consistent with previous reports that analyzed fewer dog breeds, confirming the prediction that breed barriers have led to strong genetic isolation among breeds, and are in marked contrast to the much lower genetic differentiation (typically in the range of 5-10%) found among human populations. Parker et al. (2004), citing Koskinen, Animal Genetics 34:297-301 (2003); Irion et al., Journal of Heredity 94:81-87 (2003). Variation among breeds in dogs is on the high end of the range reported for domestic livestock populations. Parker et al. (2004), citing MacHugh et al., Animal Genetics 29:333-340 (1998); Laval et al., Genet. Sel. Evol. 32:187-203 (2000).

Strong genetic differentiation among dog breeds suggests that breed membership could be determined from individual dog genotypes. In this example, a Bayesian model-based clustering algorithm is used on the microsatellite data to identify genetically distinct subpopulations or clusters on the basis of allele frequencies. Despite small among-population variance components and the rarity of "private" alleles, analysis of multilocus genotypes allows inference of genetic ancestry without relying on information about sampling locations of individuals, A model based clustering algorithm was applied by Parker et al. (2004) that, loosely speaking, identifies subgroups that have distinctive allele frequencies. This procedure, implemented in the computer program STRUCTURE, places individuals into K clusters, where K is chosen in advance but can be varied across independent runs of the algorithm, Rosenberg et al., Science 298:2381-2385 (2002). The assumption is a model in which there are K populations (where K may be unknown), each of which is characterized by a set of allele frequencies at each locus. Individuals in the sample are assigned (probabilistically) to populations or jointly to two or more populations if their genotypes indicate that they are admixed. The model does not assume a particular mutation process and it can be applied to most of the commonly used genetic markers, provided that they are not closely linked. Pritchard et al., Genetics 155:945-959 (2000). Individuals can have membership in multiple clusters, with membership coefficients summing to 1 across clusters. The algorithm attempts to identify genetically distinct subpopulations on the basis of patterns of allele frequencies. Parker et al. (2004) applied STRUCTURE to overlapping subsets of 20 to 22 breeds at a time and observed that most breeds formed distinct clusters consisting solely of all the dogs from that breed. Results of this illustrative clustering analysis showed four clusters, which Parker et al. (2004) referred to as antiquity breeds, muscular breeds, herding breeds, and hunting breeds,

Herein these same clusters are identified as Clusters I, II, III and IV respectively, to avoid confusion with phenotypically defined breed groups, such as AKC's breed groups, that may have similar names to those selected by Parker et al. (2004) for their genome-based clusters. In particular, it is pointed out that Cluster III is not coextensive with the herding group according to the AKC classification. Four Cluster III breeds (Belgian sheepdog, Belgian Tervuren, collie and Shetland sheepdog) are indeed included in the AKC herding group, but four others (Irish wolfhound, greyhound, borzoi and St. Bernard) are classified in other AKC groups. Clusters I and II likewise contain breeds that cross several AKC groups. On the other hand, Cluster IV appears to correspond closely to the AKC sporting group.

Statistical analysis of genotypes is well known in the art and is not limited to Bayesian models but may use any clustering algorithm and/or software to analyze genotype data. In various embodiments, clustering algorithms used to analyze genomic data include: hierarchical clustering (Eisen et al., Proc. Nat. Acad. Sci. 45:14863-14868 (1998)); self-organizing maps (Tamayo et al., Proc. Nat, Acad. Sci. 96:2907-2912 (1999)); k-means clustering (Tavazoie et al., Nature Genetics 22:281-285 (1999)); support vector machines (Brown et al., Proc. Nat. Acad. Sci. 97:262-267 (2000)); use of a visual display to determine the number of clusters (Eisen et al. (1998); Tamayo et al. (1999)); clustering data set leaving out one experiment at a time and then comparing the performance of different clustering algorithms using the left-out experiment (Yeung et al., Bioinformatics 17:977-987 (2001)); gap statistic estimating the number of clusters by comparing within-cluster dispersion to that of a reference null distribution (Tibshirani et al., Journal of the Royal Statistical Society 63:411-423 (2001)); hierarchical agglomerative clustering, in which two groups chosen to optimize some criterion are merged at each stage of the algorithm; use as a criterion of sum of within-group sums of squares (Ward, Journal of the American Statistical Association 58:234-244 (1963)) or the shortest distance between groups, which underlies the single-link method; iterative relocation, also called iterative partitioning, in which data points are moved from one group to another until there is no further improvement in some criterion (iterative relocation with the sum of squares criterion may be referenced as k-means clustering - see above); and graph-theoretic approaches.

Cluster analysis can also be based on probability models, Clustering algorithms based on probability models offer a principled alternative to heuristic-based algorithms. In particular, the model-based approach assumes that the data is generated by a finite mixture of underlying probability distributions such as multivariate normal distributions. According to certain examples: the Gaussian mixture model has been shown to be a powerful tool for many applications (for example, Banfield & Raftery, Biometrics 49:803-821 (1993); Celeux & Govaert, Journal of the Pattern Recognition Society 28:781-793 (1993)); problems of determining the number of clusters and of choosing an appropriate clustering method may be analyzed as a statistical model choice problem (Dasgupta & Raftery, Journal of the American Statistical Association 93:294-302 (1998); Fraley & Raftery, Journal of Classification 16:297-306 (1998)); finite mixture models have been proposed and studied in the context of clustering (Wolfe, Multivariate Behavioral Research 5:329-350 (1970); Edwards & Cavalli-Sforza, Biometrics 21:362-371 (1965); Day, Biometrika 56:463-474 (1969); Scott & Symons, Biometrics 27:387-397 (1971); Binder, Biometrika 65:31-38 (1978)); a principled statistical approach can be taken to the practical questions that arise in applying clustering methods (Fraley & Raftery, The Computer Journal 41:578-588 (1998)); and a normal mixture model band cluster analysis can be applied (Pan et al., Genome Biology 3:9.1-9.8 (2002)).

In finite mixture models, each component probability distribution may correspond to a cluster. The problems of determining the number of clusters and of choosing an appropriate clustering method can be recast as statistical model choice problems, and models that differ in numbers of components and/or in component distributions can be compared. Outliers are handled by adding one or more components representing a different distribution for outlying data. In some embodiments, methods include clustering by model based clustering, as described, for example, by Fraley & Raftery, Journal of the American Statistical Association, 97:611-631 (2002); and Yeung et al., Bioinformatics 17:309-318 (2001).

Methods combine clustering methods with a graphical representation of the primary data by representing each data point with a color or other indicia that quantitatively and qualitatively reflects the original experimental observations, The end product is a representation of complex genomic data that, through statistical organization and graphical display, allows users to assimilate and explore the data in a natural intuitive manner. In sequence comparisons, such methods may be used to infer the evolutionary history of sequences being compared, and are useful in their ability to represent varying degrees of similarity and more distant relationships among groups of closely related genes, as well as in requiring few assumptions about the nature of the data. Computed trees can be used to order genes in the tabulation of original data, so that genes or groups of genes with similar expression patterns are adjacent. The ordered table can then be displayed graphically with a representation of the tree to indicate the relationships among genes.

Methods include a model based clustering method for using multilocus genotype data to infer population structure. Examples of such methods are found in Pritchard et al. (2000); Falush et al., Genetics 164:1567-1587 (2003); Rosenberg et al. (2002); and Pritchard et al., American Journal of Human Genetics 67:170-181 (2000). Other examples of analysis of breed identification and genetic variation using microsatellite markers can be used as reported by Koskinen (2003) and Irion et al. (2003).

In various embodiments of the invention, genome-based breed clusters : : have particular nutritional needs to promote wellness, e.g., to prevent and treat disease conditions associated with each cluster. Thus, among canines, Clusters I, II, III and IV can have nutritional needs that are common to breeds within each cluster, but distinct from one cluster to another. Based on these nutritional needs, specific foods can be developed that are tailored to lifestyle, body type, activity level and other phenotypic attributes of each cluster, including incidence of particular diseases to be prevented or treated.

Within a given cluster, each breed represented can be evaluated for phenotypic characteristics common to the breed, for example, in the case of canine breeds, body size, hair shedding, trainability, and activity level based on AKC characteristics. Each characteristic can be given a ranking, e.g., by assigning a number from 1 to 3 (4 in the case of activity level) as in Table 2, and the rankings averaged across breeds within the cluster to determine an average ranking for each phenotypic characteristic. An example of data summarizing AKC breed characteristics for each of four canine breed clusters is provided in Tables 3-6.

**Table 2. AKC breed characteristic rankings**

| Ranking | Size | Shedding | Trainability | Activity Level |
|---|---|---|---|---|
| 1 | small | little | low | calm, sedate |
| 2 | medium | average | average | moderate |
| 3 | large | a lot | high | high |
| 4 | | | | very high |

**Table 3. Phenotypic analysis of canine breeds of Cluster I**

| Breed | Size | Shedding | Trainability | Activity Level |
|---|---|---|---|---|
| Basenji | medium | little | high | very high |
| Saluki | medium | little | average | moderate |
| Afghan hound | large | average | low | calm, sedate |
| Lhasa apso | small | little | high | moderate |
| Tibetan terrier | medium | average | average | moderate |
| Chow chow | medium | a lot | average | moderate |
| Pekingese | small | little | low | calm, sedate |
| Chinese shar-pei | medium | little | average | moderate |
| Shih tzu | small | little | average | high |
| Akita | large | a lot | average | very high |
| Shiba inu | medium | average | average | very high |
| Alaskan malamute | large | a lot | average | moderate |
| Siberian husky | medium | a lot | low | very high |
| Samoyed | large | a lot | average | moderate |
| Average | medium | average | average | moderate-high |

**Table 4. Phenotypic analysis of canine breeds of Cluster II**

| Breed | Size | Shedding | Trainability | Activity Level |
|---|---|---|---|---|
| Mastiff | large | Average | average | moderate |
| Bulldog | medium | a lot | average | calm, sedate |
| Boxer | medium | Average | high | high |
| Bullmastiff | large | Little | low | moderate |
| French bulldog | small | Little | average | moderate |
| German shepherd dog | large | a lot | high | very high |
| Miniature bull terrier | small | Average | average | high |
| Rottweiler | large | Average | high | high |
| Newfoundland | large | a lot | average | moderate |
| Bemese mountain dog | large | Average | high | moderate |
| Average | medium-large | Average | average | moderate |

**Table 5. Phenotypic analysis of canine breeds of Cluster III**

| Breed | Size | Shedding | Trainability | Activity Level |
|---|---|---|---|---|
| Belgian sheepdog | medium | a lot | high | high |
| Belgian Tervuren | medium | a lot | high | moderate |
| Collie | medium | a lot | high | moderate |
| Shetland sheepdog | small | a lot | high | very high |
| Irish wolfhound | large | Average | high | moderate |
| Greyhound | large | Average | average | moderate |
| Borzoi | large | a lot | average | moderate |
| St. Bernard | large | a lot | average | moderate |
| Average | medium-large | a lot | high | moderate-high |

**Table 6. Phenotypic analysis of canine breeds of Cluster IV**

| Breed | Size | Shedding | Trainability | Activity Level |
|---|---|---|---|---|
| Labrador retriever | medium | Average | high | high |
| Golden retriever | medium | Average | high | moderate |
| Cocker spaniel | medium | Average | average | moderate |
| English cocker spaniel | medium | Average | high | moderate |
| English springer spaniel | medium | Average | high | high |
| Welsh springer spaniel | medium | Average | average | very high |
| Irish water spaniel | medium | Little | high | moderate |
| Pointer | medium | Little | average | very high |
| German shorthaired pointer | medium | Little | high | high |
| German wirehaired pointer | medium | Average | high | high |
| English setter | medium | Average | average | very high |
| Gordon setter | medium | Average | average | high |
| Irish setter | medium | Average | average | high |
| Brittany | medium | Little | high | high |
| Average | medium | little-average | high | high |

In various embodiments of the invention, clusters may be evaluated for disease prevalence, to determine if specific diseases have a greater prevalence within any one cluster. Data summaries applicable for such determinations may be collected from clinical disease surveys, e.g., those conducted by veterinary colleges across the United States. Any data set of disease prevalence, whether published or not, may be used.

In an example, each breed within a cluster is evaluated for dermatitis, arthritis, obesity, eye disease, heart disease, kidney disease and hypothyroidism. It will be evident to one skilled in the art that this is not a comprehensive list of diseases and genetic disorders, and any list of diseases, genetic disorders, types of cancers or the like may be used in the present invention. For example such diseases and genetic disorders as diabetes, specific types of cancers, liver disease and gastrointestinal diseases, among many others, are not included in the present example but may be included in an evaluation. In the present example, the prevalence of disease is determined by the number of clinical cases reported, to determine if a specific breed has a high incidence of the disease. For example, if 50% or more of the breeds in a cluster have a high incidence of a particular disease type, that disease type can be considered a trait of the genetic cluster. Tables 7-10 summarize the diseases frequently diagnosed in each of four canine breed clusters.

**Table 7. Frequently diagnosed diseases of canine breeds of Cluster I**

| Breed | Dermatitis | Arthritis | Obesity | Eye Disease | Heart Disease | Kidney Disease | Hypothyroidism |
|---|---|---|---|---|---|---|---|
| Basenji | X | | | | | X | X |
| Saluki | no data | | | | | | |
| Afghan hound | X | X | | X | | | X |
| Lhasa apso | X | | | X | X | | |
| Tibetan terrier | no data | | | | | | |
| Chow chow | X | X | | X | | | |
| Pekingese | X | | | X | | | |
| Chinese shar-pei | X | | | X | | | X |
| Shih tzu | X | X | | | | X | |
| Akita | X | X | | X | | | X |
| Shiba inu | no data | | | | | | |
| Alaskan malamute | X | X | | X | | | X |
| Siberian husky | X | X | | X | | | |
| Samoyed | X | | X | X | | | X |
| Greater than 50% | X | X | | X | | | X |

**Table 8. Frequently diagnosed diseases of canine breeds of Cluster II**

| Breed | Dermatitis | Arthritis | Obesity | Eye Disease | Heart Disease | Kidney Disease | Hypothyroidism |
|---|---|---|---|---|---|---|---|
| Mastiff | X | X | | | | | X |
| Bulldog | X | X | | X | | | |
| Boxer | | X | | X | X | | X |
| Bullmastiff | X | X | | | | | X |
| French bulldog | | | | | | | |
| German shepherd dog | X | X | | X | | | |
| Miniature bull terrier | X | | | X | X | X | |
| Rottweiler | X | X | | | X | | |
| Newfoundland | X | X | | | X | | X |
| Bernese mountain dog | X | X | | | | | |
| Greater than 50% | X | X | | | | | |

**Table 9. Frequently diagnosed diseases of canine breeds of Cluster III**

| Breed | Dermatitis | Arthritis | Obesity | Eye Disease | Heart Disease | Kidney Disease | Hypothyroidism |
|---|---|---|---|---|---|---|---|
| Belgian sheepdog | no data | | | | | | |
| Belgian Tervuren | no data | | | | | | |
| Collie | X | | | X | | | |
| Shetland sheepdog | X | | X | | X | | X |
| Irish wolfhound | X | X | | | X | | X |
| Greyhound | | | | X | X | X | X |
| Borzoi | X | | | | | X | X |
| St. Bernard | X | X | | | | | |
| Greater than 50% | X | | | | | | X |

**Table 10. Frequently diagnosed diseases of canine breeds of Cluster IV**

| Breed | Dermatitis | Arthritis | Obesity | Eye Disease | Heart Disease | Kidney Disease | Hypothyroidism |
|---|---|---|---|---|---|---|---|
| Labrador retriever | X | X | X | X | | | |
| Golden retriever | X | X | X | X | | | X |
| Cocker spaniel | X | | | X | | | |
| English cocker spaniel | X | X | | X | | | |
| English springer spaniel | X | X | | X | | | |
| Welsh springer spaniel | no data | | | | | | |
| Irish water spaniel | no data | | | | | | |
| Pointer | X | X | | X | | | |
| German shorthaired pointer | X | X | | | | | |
| German wirehaired pointer | no data | | | | | | |
| English setter | X | | | X | | | X |
| Gordon setter | X | X | | X | | | X |
| Irish setter | X | X | | | | | X |
| Brittany | X | X | X | | | | |
| Greater than 50% | X | X | | X | | | |

Other methods of determining disease prevalence, incidence, frequency, or propensity are known in epidemiology, toxicology, oncology, the public health sciences, risk assessment, medicine and the like, and any such methods may be used. In various embodiments, disease prevalence is determined using an odds ratio or relative risk. In other embodiments, confounding factors and/or environmental factors are considered in determining disease prevalence. In some embodiments, each cluster is broken into groups based on age of the animal (e.g., age groups) and the determination of disease prevalence, disease incidence, disease frequency or disease propensity may be evaluated for each age group. Age groups for canines can be puppy, adult and senior, or puppy, adult, senior and geriatric. Corresponding age groups can be set up for felines and other species.

The illustrative data in Table 11 summarize characteristics of four canine breed clusters that may be used to develop specific foods tailored to meet nutritional needs for wellness, including preventing or treating conditions prevalent in the breed cluster.

**Table 11. Summary of data from Tables 3-10 for four canine breed clusters**

| Breed Cluster | Size | Shedding | Trainability | Activity Level | Frequently Diagnosed Diseases |
|---|---|---|---|---|---|
| I | medium | average | average | moderate-high | dermatitis arthritis eye disease hypothyroidism |
| II | medium-large | average | average | moderate | dermatitis arthritis |
| III | medium-large | a lot | high | moderate-high | dermatitis hypothyroidism |
| IV | medium | little-average | high | high | dermatitis arthritis eye disease |

A summary, for four illustrative canine breed clusters, of phenotypic and prevalent disease characteristics, nutrient(s) of interest with respect to each characteristic, and benefit associated with a food designed for each breed cluster and containing the nutrient(s) of interest, is shown in Tables 12-15, where EPA is eicosapentaenoic acid; DHA is docosahexaenoic acid; Mn is manganese; Zn is zinc; and n6:n3 ratio is the ratio of omega-6 to omega-3 fatty acids.

**Table 12. Cluster I summary**

| Characteristic | Nutrient(s) of interest | Benefit |
|---|---|---|
| Highly active | increase energy, enhance amino acid profile | "... promote healthy muscle and support activity level." |
| Eye disease | lutein (corn) | "... protection from free radicals that cause loss of vision." |
| Dermatitis | reduce n-6:n-3, increase linoleic acid | "... promote healthy skin, a beautiful hair coat, and reduce shedding." |
| Arthritis | EPA, methionine, Mn | "... maintain agility and mobility." |

**Table 13. Cluster II summary**

| Characteristic | Nutrient(s) of interest | Benefit |
|---|---|---|
| Arthritis | EPA, methionine, Mn | "... maintain agility and mobility." |
| Dermatitis | reduce n-6:n-3, increase linoleic acid | "... promote healthy skin, a beautiful hair coat, and reduce shedding." |
| Medium-large size | carnitine, amino acids | "... promote healthy muscle and reduce the risk of obesity." |

**Table 14. Cluster III summary**

| Characteristic | Nutrient(s) of interest | Benefit |
|---|---|---|
| Highly trainable | lipoic acid, carnitine, vitamin E, DHA | "... provide essential nutrients to enhance learning and memory." |
| Reduce hair shedding | linoleic acid, Zn | "... reduce hair shedding." |
| Highly active | increase energy, enhance amino acid profile | "... promote healthy muscle and recovery from exercise." |
| Dermatitis | reduce n-6:n-3, increase linoleic acid | "... promote healthy skin and a beautiful hair coat." |

**Table 15. Cluster IV summary**

| Characteristic | Nutrient(s) of interest | Benefit |
|---|---|---|
| Arthritis | EPA, methionine, Mn | "...maintain agility and mobility." |
| Highly trainable | lipoic acid, carnitine, vitamin E, DHA | "... provide essential nutrients to enhance learning and memory." |
| Dermatitis | reduce n-6:n-3, increase linoleic acid, gluten free | "... promote healthy skin and a beautiful hair coat." |
| Highly active | increase energy, enhance amino acid profile | "... promote healthy muscle and recovery from exercise." |
| Eye disease | lutein | "... protection from free radicals that cause loss of vision." |

As illustrated in the above example, genomic analysis identifies breed clusters that have an increased propensity for a disease, e.g., arthritis. In this example, foods may be formulated such that EPA is added to the formulation in a desired amount to help prevent and/or treat the arthritis. Without being bound by theory, EPA is believed to turn off a signal from DNA (i.e., mRNA) that makes certain degrading enzymes. For instance, in proteoglycan degradation, EPA turns off the mRNA signal to matrix metalloproteases (MMPs) and aggrecanase which are enzymes that degrade proteoglycan and may be involved in arthritis.

Similarly, other bioactive dietary components can be added to a food formulation to help prevent and/or treat other diseases to which a particular breed cluster is predisposed or to address other phenotypic characteristics of the breed cluster. For example, compositions are shown in Tables 16-19 below, which summarize compositionally the nutrients, minerals, treatments for improved health, vitamins, fatty acids, and other components for formulating a food for each of four canine breed clusters. The ingredients in each composition can be substituted for equivalent ingredients and choice may be based on cost and/or availability of ingredients at the time of production. Software programs exist in which data may be entered for ingredient components to design a food that contains the least costly components providing the required nutrients, and substitution of ingredients in such a manner is common in the pet food industry.

A method for promoting wellness of an animal can comprise (a) identifying a genome-based breed cluster to which the animal belongs; (b) selecting a nutritional formula that is matched at least in part to nutritional needs for wellness of animals of the breed cluster; and (c) feeding to the animal a food comprising BDCs in amounts and ratios dictated by the nutritional formula. Illustratively for a canine animal assigned to Cluster I, II, III or IV as defined above, the method can be directed to providing at least one of the wellness benefits identified in Table 12, 13, 14 or 15 respectively. In some embodiments, such a method is directed to providing at least two, or at least three, of the above-identified wellness benefits.

In one embodiment, a method for selecting a food further comprises identifying one or more specific zoographical attributes of the animal, In this embodiment, the food selected has a nutritional formula modified to take account of the specific zoographical attributes.

The term "zoographical attribute" as used herein refers to any and all information, whether quantitative or qualitative, that can be gathered on an animal. Sources of zoographical information can include the knowledge base of the owner, captured for example as responses to a questionnaire, veterinary records including those indicative of past and present states of wellness or disease, the animal's pedigree if it has one, biometrics (height, weight, etc.) at the time of sample acquisition, etc. Zoographical attributes illustratively include age, sex, size, weight, coat type, pedigree, reproductive history, veterinary medical history, appetite, environment-related attributes, and evident hereditary conditions and disorders of the animal.

Zoographical attributes can comprise one or more attributes relating to genotype. Examples of such attributes include, without limitation, the breed of the animal, whether pedigreed, registered by a body such as AKC or otherwise; pedigree if known; in the case of animals of mixed breed, the breed heritage of the animal including the breed(s) of its parents and, if available, ancestors of earlier generations; sex; coat type (e.g., long, short, wiry, curly, smooth) and coloration; evident hereditary conditions and disorders; etc.

Zoographical attributes can comprise one or more attributes relating to physiological condition. Examples of such attributes include, without limitation, age (chronological and, if determinable, physiological); weight; dimensions (e.g., height at shoulder, length of legs, length of back, etc.); veterinary medical history; reproductive history, including whether neutered, number and size of litters, etc.; present wellness or disease state and any recent changes therein, including any condition or disorder diagnosed, and any symptoms whether or not diagnosis has been made; presence of parasites, including fleas; appetite and any recent changes therein; physical activity level; mental acuity; behavioral abnormalities; disposition (e.g., timid, aggressive, obedient, nervous); etc.

Zoographical attributes can further relate to aspects of the environment in which the animal lives. Such aspects include, without limitation, climate, season, geographical location and habitation. For example, it can be material to developing a food composition for a companion animal to know whether the animal lives in a warm or dry climate, or an arid or humid climate; whether it is currently spring, summer, autumn, or winter; whether the animal is housed indoors or outdoors; whether the animal is in a home, a boarding kennel, a place of work (e.g., in the case of guard dogs, police dogs, etc.) or some other habitat; whether it is housed alone or with other animals; whether it lives in an urban or rural area; zip code, state and/or country of occupancy; whether and to what extent its habitat is affected by pollutants (e.g., tobacco smoke); etc.

The breed cluster and specific zoographical attributes of the animal can be identified from input data provided by an owner. Such input data can be entered by the owner via a user interface, that can comprise, e.g., a computer, a touch-screen video terminal, a touch-tone telephone or a voice-activated system.

In another embodiment, a method for selecting a food further comprises identifying one or more specific wellness attributes of the animal. In this embodiment, the food selected has a nutritional formula modified to take account of the specific wellness attributes. Any of the wellness attributes mentioned hereinabove can be included. Wellness attributes can be identified from input data provided by an owner and/or a veterinary professional. Such input data can comprise diagnostic data from a biofluid or tissue sample obtained from the animal.

A biofluid or tissue sample useful herein can be any such sample that is amenable to analysis for diagnostic purposes. Biofluids that can be sampled include excreta (feces and urine), blood, saliva, amniotic fluid, etc. Tissue samples can be obtained for example by biopsy, by surgical removal (e.g., during surgery being conducted for other purposes), by cheek swab or by pulling a few hairs.

Optionally, a tissue or biofluid sample can be used for genomic analysis, to help assign the animal to an appropriate breed cluster. This can be especially helpful where the animal is of unknown or mixed breed or genetic heritage. In this case, the sample must be capable of providing DNA or RNA, in a quantity that may or may not need amplification, e.g., through PCR techniques.

Single nucleotide polymorphisms (SNPs) can be particularly useful in assigning an animal to a breed cluster. Some SNPs are breed-specific. The term "breed-specific SNP", as used herein, means a SNP that can be used to distinguish between different breeds or to determine breed inheritance, either alone or in combination with other SNPs. Such a breed-specific SNP may be unique to one breed. Alternatively, a breed-specific SNP may be present in a plurality of breeds, but its presence in combination with one or more other breed-specific SNPs can be used to determine an animal's breed inheritance. In one embodiment of the invention, a SNP is used that is present in substantially all dogs of one breed and is absent in substantially all dogs of other breeds. The breed-specificity of a SNP is typically assessed in a sample population that is representative of a particular breed. Such a sample population typically consists only of purebred animals. The sample population typically comprises at least about 4 animals per breed, e.g., at least about 20, at least about 100, at least about 400, at least about 1000 or at least about 10,000 animals of one breed.

In the case of canines, a breed-specific SNP is typically present in at least about 70%, at least about 80%, or at least about 90% of the sample population of a breed, and is preferably present in at least about 95%, more preferably at least about 99% of the sample population. The breed-specific SNP is typically absent in substantially all dogs of sample populations of other breeds. For example, a breed-specific SNP may be present in no more than about 30%, no more than about 20%, or no more than about 10% of a sample population of another breed, preferably no more than about 5%, more preferably no more than about 1% of the sample population. In various embodiments, the SNP is present in at least about 95% of a sample population of a breed and in no more than about 5% of a sample population of dogs of any other breed. In some embodiments, the breed-specific SNP is unique to a breed, i.e., it is present in 100% of a sample population which is representative of that breed and is entirely absent from a sample population which is representative of any other breed.

In a further embodiment of the invention, a breed-specific SNP can be used to distinguish one breed in a panel of breeds from the other breeds in the panel. In such an embodiment, the SNP is thus specific for one of the breeds in the panel. In other embodiments, the SNP may be found in more than one breed. A SNP that is specific for two or more breeds within a breed cluster can be used to distinguish those particular breeds from other breeds in the breed cluster.

In some embodiments, an animal's breed is defined not by a single SNP, but by a combination of SNPs present in the animal's genome. Accordingly, in such embodiments the breed inheritance of an animal may be identified from a combination of the nucleotides present at two or more SNP positions. Each breed may therefore be defined by a rule or set of rules based on the combination of nucleotides found at these positions. In some cases, in order to define a breed, it may be necessary to provide one or more rules which specify the nucleotide at each of a plurality of SNP positions. In the case of a canine, in order to identify the canine's breed inheritance, typically at least two different SNP positions are typed. Typing generally comprises determining the nucleotide present at any given SNP position.

The term "breed inheritance" is used herein to describe the breed ancestry of an animal, namely the one or more breeds that have contributed to the animal's genome. Therefore, in the case of a purebred dog, breed inheritance typically corresponds to the breed of the dog. Accordingly, in one embodiment, the nucleotide present at each of one or more SNP positions in the dog's genome can be used to determine breed inheritance of the dog. In the case of a crossbred or outbred animal, the term "breed inheritance" may relate to a plurality of breeds that are represented in the animal's lineage. "Breed inheritance" may further be used to describe the proportions or relative contribution of each breed in the ancestry of an outbred animal.

In some embodiments, a dog that is tested for breed inheritance may be a crossbred or outbred dog. A crossbred dog is the offspring of two purebred dogs of different breeds. An outbred dog (which also may be known as a mongrel, mixed-breed dog or mutt) is of unknown parentage, or the result of a combination cf three or more breeds over two or more generations. The breeds that contribute to an outbred animal's breed inheritance may be from within one breed cluster or from different breed clusters.

In some embodiments, a crossbred or outbred dog may have its breed inheritance analyzed based on genetic material obtained from a tissue or biofluid sample, to identify one or more breeds that are represented in the dog. Optionally, a determination can then be made as to the percentage contribution of each breed to the dog's lineage. If substantially all of the dog's ancestry comes from breeds in a single cluster, the dog can be assigned to that cluster. However, if the ancestral breeds of the dog are in two or more clusters, further analysis may be needed to assign the dog to a cluster. In some embodiments, the breed cluster to which a crossbred or outbred dog belongs may be determined using statistical analysis techniques. Certain crossbred dogs which are largely represented in the overall canine population may be added as specific members of a breed cluster. In one embodiment, SNPs may be used as a basis for determining breed clusters. In another embodiment, linkage disequilibrium may be used as a basis for determining breed clusters, e.g., as shown by Sutter et al., Genomic Research 14:2388-2396 (2004).

The sequences of breed-specific SNPs may be stored in an electronic format, e.g., in a computer database. Accordingly, the invention provides a database comprising genomic information relating to breed-specific SNPs. The database may include further information about a SNP, e.g., the level of association of the SNP with a breed or the frequency of the SNP in the breed. In various embodiments, the database further assigns each of the breed-specific SNPs to a specific breed cluster. A database as described herein may be used to assign an animal to a breed cluster. Such a determination may be carried out by electronic means, e.g., by using a computer system. Typically, the determination is carried out by inputting genetic data from an animal to a computer system; comparing the genetic data to a database comprising information relating to breed-specific SNPs; and, on the basis of the nucleotide present at each of one or more breed-specific SNP positions, identifying the breed inheritance of the animal and assigning the animal to a breed cluster. In the case of a dog, the method comprises inputting data relating to breed-specific SNPs present in the dog to a computer system; comparing these data to a database which comprises information relating to breed-specific SNPs in different breeds and/or breed clusters; and on the basis of this comparison assigning the dog to a breed cluster.

A method of the invention for preparing a food for an animal comprises identifying a breed cluster to which the animal belongs and establishing a set of nutritional needs for wellness characteristic of that breed cluster, as set forth above. The method further comprises selecting a nutritional formula that is matched at least in part to those nutritional needs, and compounding ingredients that provide BDCs in amounts and ratios dictated by the nutritional formula, to provide the food.

The nutritional formula can take the form of a substantially complete food formula, including basic nutrients such as protein, carbohydrate, lipid and fiber, as well as the BDCs required to satisfy the particular nutritional needs for wellness associated with the breed cluster to which the animal belongs. Alternatively, the nutritional formula can take the form of a supplement formula providing BDCs in amounts and ratios meeting nutritional needs for wellness when added to a base food.

Examples of BDCs that are chemical entities include without limitation: amino acids; simple sugars; complex sugars; medium-chain triglycerides (MCTs); triacylglycerides (TAGs); n-3 (omega-3) fatty acids including α-linolenic acid (ALA), eicosapentaenoic acid (EPA) and docosahexaenoic acid (DHA); n-6 (omega-6) fatty acids including linoleic acid, γ-linolenic acid (GLA) and arachidonic acid; choline sources such as lecithin; fat-soluble vitamins including vitamin A and precursors thereof such as carotenoids (e.g., β-carotene), vitamin D sources such as vitamin D₂ (ergocalciferol) and vitamin D₃ (cholecalciferol), vitamin E sources such as tocopherols (e.g., α-tocopherol) and tocotrienols, and vitamin K sources such as vitamin K₁ (phylloquinone) and vitamin K₂ (menadione); water-soluble vitamins including B vitamins such as riboflavin, niacin (including nicotinamide and nicotinic acid), pyridoxine, pantothenic acid, folic acid, biotin and cobalamin; and vitamin C (ascorbic acid); antioxidants, including some of the vitamins listed above, especially vitamins E and C; also bioflavonoids such as catechin, quercetin and theaflavin; quinones such as ubiquinone; carotenoids such as lycopene and lycoxanthin; resveratrol; and α-lipoic acid; L-carnitine; D-limonene; glucosamine; S-adenosylmethionine; and Chitosan.

With respect to the inclusion of amino acids in the above illustrative list of BDCs, it will be noted that almost all foods contain protein, which typically supplies all essential amino acids. However, the protein content of a food does not necessarily supply essential amino acids in proportions that are optimal for wellness of particular animals, thus supplementation with one or more amino acids, or with protein sources rich in such amino acids, can be desirable.

Similar considerations apply in the case of simple and complex sugars that are BDCs and may or may not be components of the carbohydrate fraction of a food, and certain fatty acids, including n-3 and n-6 fatty acids, that are BDCs and may or may not be components of the lipid fraction of a food.

Illustrative botanicals that can be useful as BDCs include, without limitation, aloe vera, dong quai, echinacea, evening primrose, flaxseed, garlic, ginger, ginkgo biloba, ginseng, green tea, soy, turmeric, wheat grass and yerba mate.

The food prepared by the method can be substantially nutritionally complete, or it can constitute a supplement adapted for feeding in conjunction or in mixture with a base food. Where the food prepared is a supplement, the method can further comprise compounding the supplement with a base food prior to packaging. Alternatively, the supplement can be supplied to the owner of the animal, for addition to a base food at the point of feeding to the animal.

The ingredients providing the BDCs can be selected by an algorithm. Algorithms for formulating food compositions based on a nutritional formula are well known in the art. Such algorithms access a data set having analysis of various ingredients and draw on that data set to compute the amounts of such ingredients in a food composition having the desired nutritional formula.

Optionally the data set on which the algorithm draws further includes cost data for the various ingredients, and the algorithm incorporates a routine to include cost as a criterion in selection of ingredients. This can enable a food to be prepared at advantageous overall cost at lowest cost consistent with providing the desired nutritional formula. Other criteria can be built in if desired. For example, ingredients can be identified as "organic" or otherwise, so that if an "organic" food product is desired only "organic" ingredients are selected. Examples of "organic" ingredients include any agricultural product that is produced and handled in accordance with requirements specified by the U.S. Food & Drug Administration (FDA), as set forth in 7 CFR Sec. 205.101, Secs. 205.202-207, Secs. 205.236-239, Sec. 205.101, Secs. 205.270-272 and all other applicable requirements of 7 CFR part 205.

In one embodiment, the food composition can be selected from a range of pre-existing options, e.g., an existing food product line, to best fit or match the nutritional formula derived by practice of the invention. For example, an algorithm can be used that compares a computed food composition or nutritional formula with those of available products, and selects the product coming closest to matching that composition or formula.

In another embodiment, a food is manufactured according to the composition derived from an algorithm as set forth above. Such manufacture can be offline, i.e., not controlled by a computer-aided system, or in part or in whole under the control of, and/or driven by, an extension of a computer-aided system that generates the nutritional formula and computes a composition for the food as described above.

The product thus manufactured can be a complete food or a supplement adapted for addition to or mixing with a base food to form a complete food. The product can be liquid, semi-solid or solid; if solid, it can be moist (e.g., a retortable moist pet food), semi-moist or dry (e.g., a kibble). A supplement can be designed for use, e.g., as a gravy to accompany a base food, or as a coating for a base kibble.

Suitable computer-controlled apparatus for manufacturing a food product having a defined composition is known in the art. Illustratively, apparatus substantially as described in U.S. Patent No. 6,493,641 can be used.

Optionally, the food, once prepared according to a method of the invention, is packaged in a suitable container. For example, a moist food can be packaged in a can, a jar or a sealed pouch; a dry food can be packaged in a bag, a box, or a bag in a box. This step can, if desired, also be under control of a computer-aided system.

A computer-aided system as contemplated herein can be further harnessed to print a label or package insert for the food product, having any or all information required by governmental regulations and by customary commercial practice. For example, the label or package insert can include a list of ingredients and/or a guaranteed analysis.

Food manufacture, including packaging and labeling, can occur at a conventional manufacturing site such as a factory. Alternatively, it can be convenient to arrange for manufacture of the food to take place more locally to the end-user, e.g., at a point of sale at a distributor's or retailer's premises, such as a pet food store. In one embodiment the food composition is prepared at a distribution site and delivered to the end-user, e.g., in response to an order placed by the end-user, such as by telephone or via a website accessed through the internet.

The food composition can, in one embodiment, be prepared by a retailer or compounder on receipt of a prescription from a veterinary physician or dietician setting forth the nutritional formula, The prescription optionally comprises a coupon validated for use in payment at least in part for the food, or entitling the bearer of the coupon to a discount or rebate on purchase of the food.

Also disclosed are food compositions for animals of particular genotype as defined by breed cluster, Illustrative food compositions of the invention include, in the case of canine animals, foods for a canine of Clusters I, II, III and IV. In some embodiments, the food composition is for a canine of a breed cluster that comprises breeds from two or more AKC breed groups.

For a canine of Cluster I, a food suitable as a substantially nutritionally complete diet illustratively has a nutritional formula that comprises, by weight on a dry matter basis, about 28% protein, about 18% fat, about 51% carbohydrate including fiber, about 0.2% EPA, about 1.5% methionine and about 100 ppm manganese, with a weight ratio of omcga-6 to omega-3 fatty acids of about 6:1. An example of such a food is shown in Table 16.

**Table 16. Food composition for a canine of Cluster I**

| Ingredient | % of food | Ingredient | % offood |
|---|---|---|---|
| Corn | 51.240 | Vitamin E | 0.200 |
| Poultry By-Product Meal | 18.210 | Vitamin Premix | 0.126 |
| Soybean Meal | 15.000 | Taurine | 0.100 |
| Chicken Fat | 8.953 | Mineral Mix | 0.040 |
| Pal Enhancer | 2.000 | Manganese Sulfate | 0.023 |
| Soybean Oil | 1.000 | L-Tryptophan | 0.017 |
| Fish Oil | 1.000 | Crude protein | 28.400 |
| DL-Methionine | 0.894 | Crude fat | 18.400 |
| Non-Iodized Salt | 0.642 | EPA | 0.200 |
| Choline Chloride | 0.285 | Methionine | 1.500 |
| L-Carnitine | 0.270 | Manganese | 0.010 |
| | | Omega-6:omega-3 ratio | 6 |

For a canine of Cluster II, a food suitable as a substantially nutritionally complete diet illustratively has a nutritional formula that comprises, by weight on a dry matter basis, about 28.5% protein, about 16.5% fat, about 53% carbohydrate including fiber, less than about 0.2% EPA , about 1.5% methionine, about 100 ppm manganese and about 300 ppm carnitine. An example of such a food is shown in Table 17.

**Table 17. Food composition for a canine of Cluster II**

| Ingredient | % of food | Ingredient | % of food |
|---|---|---|---|
| Corn | 53.393 | Vitamin Premix | 0.126 |
| Poultry By-Product Meal | 18.136 | Taurine | 0.100 |
| Soybean Meal | 14.628 | L-Threonine | 0.081 |
| Chicken Fat | 7.245 | L-Tryptophan | 0.058 |
| Pal Enhancer | 2.000 | Potassium Chloride | 0.050 |
| Soybean Oil | 1.000 | Mineral Mix | 0.034 |
| Fish Oil | 1.000 | Manganese Sulfate | 0.023 |
| DL-Methionine | 0.899 | Crude protein | 28.500 |
| Salt Iodized | 0.280 | Crude fat | 16.500 |
| L-Camitine | 0.270 | EPA | <6.0 |
| Choline Chloride | 0.240 | Methionine | 1.500 |
| L-Lysine | 0.236 | Manganese | 0.010 |

For a canine of Cluster III, a food suitable as a substantially nutritionally complete diet illustratively has a nutritional formula that comprises, by weight on a dry matter basis, about 30% protein, about 26% fat, about 40% carbohydrate including fiber, about 0.14% DHA, about 4.8% linoleic acid and about 300 ppm carnitine. An example of such a food is shown in Table 18.

**Table 18. Food composition for a canine of Cluster III**

| Ingredient | % of food | Ingredient | % of food |
|---|---|---|---|
| Corn | 40.649 | Vitamin Premix | 0.211 |
| Poultry By-Product Meal | 23.252 | Vitamin E Oil | 0.200 |
| Chicken Fat | 15.522 | Taurine | 0.100 |
| Soybean Meal | 13.263 | Potassium Chloride | 0.050 |
| Pal Enhancer | 2.000 | Mineral Mix | 0.050 |
| Soybean Oil | 1.000 | L-Tryptophan | 0.030 |
| Fish Oil | 1.000 | Manganese Sulfate | 0.023 |
| DL-Methionine | 0.875 | Crude protein | 30.000 |
| Non-Iodized Salt | 0.610 | Crude fat | 26.000 |
| Flaxseed | 0.600 | Lipoic acid | 0.015 |
| Choline Chloride | 0.295 | DHA | 0.140 |
| L-Carnitine | 0.270 | Linoleic acid | 4.820 |
| | | Carnitine | 0.030 |

For a canine of Cluster IV, a food suitable as a substantially nutritionally complete diet illustratively has a nutritional formula that comprises, by weight on a dry matter basis, about 28.5% protein, about 16.5% fat, about 53% carbohydrate including fiber, about 0.14% DHA, about 4.8% linoleic acid, about 0.875% methionine, about 300 ppm carnitine and about 100 ppm manganese. An example of such a food is shown in Table 19.

**Table 19. Food composition for a canine of Cluster IV**

| Ingredient | % of food | Ingredient | % of food |
|---|---|---|---|
| Corn | 52.946 | Taurine | 0.100 |
| Poultry By-Product Meal | 18.151 | Potassium Chloride | 0.050 |
| Soybean Meal | 14.981 | Mineral Mix | 0.034 |
| Chicken Fat | 7.006 | Manganese Sulfate | 0.023 |
| Pal Enhancer | 2.000 | L-Tryptophan | 0.015 |
| Soybean Oil | 1.000 | Crude protein | 28.500 |
| Fish Oil | 1.000 | Crude fat | 16.500 |
| DL-Methion ine | 0.895 | Lipoic acid | 0.015 |
| Flaxseed | 0.600 | DHA | 0.140 |
| Salt Iodized | 0.280 | Linoleic acid | 4.820 |
| L-Carnitine | 0.270 | Carnitine | 0.030 |
| Choline Chloride | 0.240 | EPA | 0.200 |
| Vitamin Premix | 0.208 | Methionine | 1.500 |
| Vitamin E | 0.200 | Manganese | 0.010 |

Age is a factor in the nutrition and health of any human or animal. In some embodiments, the clusters are broken into groups based on the age of the animal (age groups) and the determination of disease prevalence, disease incidence, or disease propensity may be made for each age group. The "chronological age" of an animal is the actual time elapsed (e.g., in years or months) since birth. The "physiological age" of an animal is an estimate of the average chronological age of animals of similar breed exhibiting the same age-related physiological condition (mobility, mental acuity, dental wear, etc.) as the animal.

The breed clusters and phenotypic information for each cluster may be used in designing pharmaceutical compositions for an animal; in designing wellness programs for an animal; in programs to determine if supplements are needed in an animal's diet as well as the types and quantities of supplements that should be recommended; in designing therapeutic regimens for an animal, e.g., a regimen that includes an exercise program to prevent onset of a chronic condition that is prevalent in the cluster; or to formulate foods that comprise BDCs that may be important in treating and/or preventing a disease or a genetic disorder.

Formulations of food containing BDCs for prevention and/or treatment of diseases or genetic disorders are, in some embodiments, available by prescription.

In various embodiments, foods are formulated to optimize realization of the genetic potential of an animal.

Various embodiments of the invention include creation of a nutritional formula matrix that includes genotype as one of the axes. An axis perpendicular to the genotype axis can be age or age group, or, in other embodiments, disease prevalence or physical attributes. It will be evident to those skilled in the art that the perpendicular axis may represent any one or more of an infinite set of different phenotypic, age, disease, ingredient requirements (such as organic, hypoallergenic, vitamin enriched, cost point, etc.), other characteristics and the like.

An illustrative method of constructing a formula matrix for an animal species is shown in Figure 1. A clustering algorithm is used to classify breeds into genome-based breed clusters. The clusters are then characterized by nutritional requirements and by disease propensity and consequent need for prevention and/or treatment of diseases. In this illustrative method, age groups of the animal species are also characterized by nutritional requirements and need for prevention and/or treatment of diseases. Nutritional requirements and disease prevention and/or treatment needs, as affected by breed cluster and age, determine ingredients that should be included in food formulas to provide BDCs satisfying such nutritional requirements and disease prevention and/or treatment needs. Cost is an optional additional criterion in selecting ingredients. In this way, a matrix of food formulas, having breed cluster as a first dimension and age as a second dimension, is created. Figure 1 shows each cell of the matrix occupied by a food formula; however, it will be understood that not every cell of the matrix must be occupied, and that a given cell can have more than one food formula. A food formula occupying any cell can, in some embodiments, be a formula for a supplement that can be added either by a compounder or by an owner to a base food. The information used to create the matrix, including that relating to clusters, age groups, disease propensity, prevention and treatment, nutritional requirements, BDCs, ingredients, etc., can be stored in one or more databases and algorithms can draw information from such databases in creating the formula matrix.

In a related embodiment, a method is provided for constructing a matrix of food compositions for an animal species not having recognized breeds. The method comprises identifying a plurality of genotypes within the species, classifying the genotypes into clusters based on genomic analysis, associating each cluster with nutritional needs for wellness, and selecting a blend of food ingredients satisfying these nutritional needs for each cluster, to construct the matrix of food compositions. As in other embodiments, any food composition in the matrix can optionally be a supplement composition that can be added either by a compounder or by an owner to a base food.

In one embodiment, the number of food compositions corresponds to the number of clusters. For example, if canine breeds are classified into four breed clusters, e.g., Clusters I, II, III and IV, there can be one food for each of these clusters.

In other embodiments, the matrix has at least two dimensions, one of which corresponds to breed cluster. A second dimension can correspond to age of the animal, thus the method can further comprise defining age groups within the species. A two-dimensional food matrix can be generated, comprising a food composition adapted for the nutritional needs of each age group within each cluster.

In still further embodiments, the matrix has more than two dimensions. For example, a first dimension can correspond to breed clustcr, a second to age or age group, and a third to particular health or wellness state, e.g., body condition.

As in other embodiments, the food ingredients can be selected based on criteria that include cost, such that the matrix of food compositions can be prepared at advantageous overall cost.

Further disclosed is a consumer communication apparatus. Such apparatus helps an owner understand which genotypic cluster his/her animal, e.g., canine, belongs to. Such apparatus can include any one or more of a variety of point of sale displays which are well known in the art of marketing. In various embodiments, a consumer communication apparatus includes a computer kiosk, at a point of sale or elsewhere, which allows the owner to input information, e.g., on a touch screen, including for example breed and age, and in certain embodiments other information may be required to be input. Based on the input information, the computer identifies an appropriate food formulation, or if only one formulation is appropriate, the correct food formulation, for the animal. In various embodiments, the computer kiosk provides frequently asked questions along with answers. In other embodiments, the computer kiosk provides a tutorial or primer on the science that is involved in development of the food formulations. In various embodiments, the computer kiosk may be used to market new products and/or new technology. In other embodiments, the computer kiosk may be used to collect survey information from consumers.

In some embodiments, the kiosk runs a web page or a group of web pages over the Internet. In other embodiments, an owner inputs information and/or receives information via web pages on a computer. In such embodiments, the web pages on the computer may perform one or more functions in a similar fashion to the kiosk described above. In still other embodiments, an owner responds to a questionnaire in a written format and the questionnaire is then input to a system that determines the most appropriate food formulation for a particular animal. In still other embodiments, an owner responds to questions orally and such oral responses may be recorded electronically or in a paper format by a health care professional and such response is input to a system that determines the most appropriate food formulation for a particular animal, In some embodiments, the oral responses are recorded electronically by a computer and such responses are converted electronically and placed through the system to determine an appropriate food formulation for a particular animal. In various embodiments, which may use any of the above described apparatus, an owner does not know which breed his or her animal belongs to, and thus cannot readily determine which breed cluster the animal fits. In such embodiments, a questionnaire using any of the above apparatus and/or methods may be used. In such questionnaire, the owner may be asked a series of questions related to phenotypic characteristics of the animal and the responses to the questions are input to the system for determination of a breed cluster that is the best fit for the animal. In such embodiments, the determination of breed cluster can then be input to a system that determines the most appropriate food formulation for their animal,

In other embodiments, a point of sale display may be similar to one found in an auto parts store or an auto parts aisle of a department store. Such point of sale displays are commonly used for headlights, batteries, brake lights, interior lights, windshield wipers, and other commonly purchased automotive maintenance items. In the present instance, point of sale display items may include flip charts that can help identify the proper formulation for an animal. In other embodiments, the point of sale display may comprise a small microprocessor, as is common in auto parts aisles, that asks for the breed and the age of the animal, then the microprocessor outputs a proper formulation for the animal. In other embodiments, the point of sale display can include charts and displays that include some of the scientific features of the formulations based on genotype clusters. In various embodiments, graphics may be included on the food packaging, e.g., bags, indicating a particular genotype cluster for which the food is designed or appropriate. Such graphics may be displayed in charts, computers, microprocessors, square or round flip charts and the like so that identification of the appropriate cluster is easily found on the bag or other packaging. In other embodiments, colors may be used for identification purposes. In other embodiments, graphics or colors may be used to identify food designed or appropriate for different age groups. In various embodiments, the bag or other packaging carries a listing of breeds that are included in the particular genotype cluster for which a food is designed or appropriate. In various embodiments, the bag or other packaging carries instructions for feeding based on body weight.

In any of the above embodiments, the consumer communication apparatus optionally generates a coupon validated for use in payment at least in part for the food, or entitling the bearer of the coupon to a discount or rebate on purchase of the food.

In another embodiment, a method further comprises downloading a code representing the nutritional formula to a readable medium, e.g., a computer-readable medium such as a printed barcode, a printed numerical code, a card, a memory, a disk or a chip, optionally a chip adapted for implantation in the animal.

According to this embodiment, an owner at a point of sale terminal can enter a code representing a nutritional formula previously selected for a specific animal, e.g., by swiping a card or scanning a chip containing such a code. A computer-aided mixing apparatus, e.g., a mixing and vending apparatus located at the point of sale, then prepares a food composition based on the nutritional formula thus encoded, and delivers it to the owner. The card or chip optionally contains further code permitting automatic payment for the food.

A computer-aided system for designing a nutritional formula for an animal is a further embodiment of the invention. The system comprises, on one to a plurality of user-interfaceable media, (a) a data set, herein referred to as a first data set, relating a plurality of breed clusters to genome-related attributes of each breed cluster; and (b) an algorithm, herein referred to as a first algorithm. This algorithm is capable, while drawing on the first data set, of (i) processing input data on one or more genome-related attributes of the animal to define a breed cluster to which the animal can be allocated, and (ii) formulating a nutritional formula appropriate to nutritional needs of the breed cluster. The computer aided system of the present invention is for implementing the method of the present invention, and is further described in the claims.

Genome-related attributes populating the first data set and constituting the input data can include one or more of breed, breed inheritance and genetic markers, If the animal's breed is known and is not mixed, and the first data set includes a list of breeds for each breed cluster, the first algorithm can readily identify the animal's breed cluster from its breed, no other information being necessary. Similarly, for an animal of mixed breed, if its breed inheritance is known, the algorithm can derive a best-fit breed cluster based on breed inheritance input data. Alternatively or in addition, input data can include one or more genetic markers that individually or collectively are indicative of a breed cluster. Such genetic markers, e.g., SNPs, can be derived by analysis of a biofluid or tissue sample obtained from the animal.

In one embodiment, the system further comprises (c) a second data set recording phenotypic attributes characteristic of each breed cluster; and (d) a third data set relating to effects of BDCs (i) on such phenotypic attributes, as modified by specific zoographical attributes, and optionally (ii) on specific wellness attributes of individual animals. According to this embodiment, the first algorithm is further capable, while drawing on the second and third data sets, of processing input data on one or more zoographical attributes and optionally one or more wellness attributes of the animal to derive the nutritional formula, The nutritional formula is not only appropriate to nutritional needs of the breed cluster but further promotes wellness of the animal by taking into account zoographical attributes such as age and optionally specific wellness attributes such as an existing disease condition.

The system optionally further comprises a user interface, The first, second, and third data sets can reside in one database or in a plurality of separate databases. The zoographical attributes acting as modifiers in the third data set can include any of those mentioned hereinabove. The first algorithm is optionally capable of processing input data that comprise diagnostic data from a biofluid or tissue sample obtained from the animal.

The system can, if desired, further comprise (e) a fourth data set relating to contents of BDCs in food ingredients and, optionally, costs of these ingredients; and (f) a second algorithm capable of selecting food ingredients from the fourth data set to define a food composition having a nutritional formula as defined by the first algorithm. This second algorithm optionally takes account of costs of ingredients to define a food composition having advantageous overall cost. The system can further comprise a computer-controlled mixing system capable of preparing the food composition defined by the second algorithm.

A computer-aided system as provided herein can further comprise a packaging system capable of placing a metered amount of the food composition in a suitable container, and/or a labeling system capable of printing a label or package insert with output data defining the breed cluster and optionally other attributes of the animal for which the food composition has been prepared, and providing information on the nutritional formula and/or ingredients of the food composition.

A kit may comprises a food prepared by a method as described herein, a food supplement, a food, and optionally a means of communicating information and/or instructions on adding the food supplement to the base food and feeding the resulting supplemented food to an animal. The supplement and the food are typically presented in separate containers, which can be co-packaged or distributed in separate packages, The communicating means can illustratively take the form of a label or package insert. Alternatively or in addition, the communicating means can comprise a brochure, advertisement, computer-readable digital or optical medium such as a diskette or CD, an audio presentation on an audiotape or CD, a visual presentation on a videotape or DVD, and/or one or more pages on a website.

The examples and other embodiments described herein are exemplary and are not intended to be limiting in describing the full scope of systems, and methods of this invention.

The words "comprise", "comprises", and "comprising" arc to be interpreted inclusively rather than exclusively.

## Claims

1. A method of formulating and preparing a food composition for a canine comprising:
(a) identifying a genome-based breed cluster to which the canine belongs;
(b) formulating a food composition for the canine having a nutritional formula matched at least in part to nutritional needs of canines in the breed cluster; and
(c) preparing a food composition for the canine;
wherein the genome-based breed cluster is selected from the group consisting of Cluster I, Cluster II, Cluster III and Cluster IV as defined in Parker et al., Science 304: 1160-1164, 2004,
and wherein steps (a) and (b) are performed using a computer-aided system.

2. The method of Claim 1 wherein the nutritional needs are based at least in part on one or more phenotypic attributes characteristic of the breed cluster selected from the group consisting of size, coat type, trainability, activity level and prevalence of and predisposition to diseases.

3. The method of Claim 1 further comprising identifying one or more specific zoographical attributes of the canine, selected from the group consisting of age, sex, size, weight, coat type, pedigree, reproductive history, veterinary medical history, appetite, environment-related attributes, and evident hereditary conditions and disorders; wherein the food selected has a nutritional formula modified to take account of the specific zoographical attribute(s).

4. The method of Claim 3 wherein the breed cluster and the specific zoographical attribute(s) of the canine are identified from input data provided by an owner of the canine.

5. The method of Claim 4 wherein the input data are entered by the owner via a user interface comprising a computer, a touch-screen video terminal, a touch-tone telephone and/or a voice-activated system.

6. The method of Claim 1 further comprising identifying one or more specific wellness attributes of the canine selected from the group consisting of disease states, states of parasitic infestation, hair and skin condition, sensory acuteness, dispositional and behavioral attributes, and cognitive function; wherein the food selected has a nutritional formula modified to take account of the specific wellness attribute(s).

7. The method of Claim 6, wherein the specific wellness attribute(s) of the canine are identified from input data provided by an owner of the canine and/or a veterinary professional.

8. The method of Claim 7 wherein the input data comprise diagnostic data obtained from a biofluid or tissue sample from the canine.

9. The method of Claim 1 further comprising compounding ingredients that provide bioactive dietary components in amounts and ratios consistent with the nutritional formula, to prepare the food.

10. The method of Claim 9 wherein the food constitutes a supplement adapted for feeding in conjunction or in mixture with a base food.

11. The method of Claim 9 wherein the food is prepared at a manufacturing site; at a point of sale; or at a distribution site and delivered to an owner of the canine.

12. The method of Claim 10 wherein the food is supplied by a retailer or prepared by a compounder on receipt of a prescription from a veterinary physician or dietician setting forth the nutritional formula.

13. The method of Claim 12 wherein the prescription comprises a coupon validated for use in payment at least in part for the food, or entitling a bearer of the coupon to a discount or rebate on purchase of the food.

14. A computer-aided system comprising one to a plurality of user-interfaceable media for implementing the method of any preceding claim, comprising :
(a) a first data set relating a plurality of breed clusters to genome-related attributes of each breed cluster, wherein the genome-based breed clusters are Cluster I, Cluster II, Cluster III and Cluster IV as defined in Parker et al., Science 304: 1160-1164, 2004; and
(b) a first algorithm capable, while drawing on the first data set, of (i) processing input data on one or more genome-related attributes of the canine to define a breed cluster to which the canine can be allocated, and (ii) formulating a food composition for the canine having a nutritional formula matched at least in part to nutritional needs of canines in the breed cluster.

15. The system of Claim 14 wherein the input data comprise one or more genetic markers individually or collectively indicative of a breed cluster, the one or more genetic markers being derived by analysis of a biofluid or tissue sample obtained from the canine.

16. The system of Claim 14, further comprising:
(c) a second data set recording phenotypic attributes characteristic of each breed cluster; and
(d) a third data set relating to effects of bioactive dietary components (i) on the phenotypic attributes as modified by specific zoographical attributes, and optionally (ii) on specific wellness attributes of individual canines; wherein the first algorithm is further capable, while drawing on the second and third data sets, of processing input data on one or more zoographical attributes and optionally one or more wellness attributes of the canine to formulate a food composition for the canine having a nutritional formula matched at least in part to nutritional needs of canines in the breed cluster, and optionally,
wherein the food composition is suitable for promoting wellness of the canine.

17. The system of Claim 14 wherein the zoographical attributes acting as modifiers in the third data set comprise one or more attributes selected from the group consisting of age, sex, size, weight, coat type, pedigree, reproductive history, veterinary medical history, appetite, environment-related attributes, and evident hereditary conditions and disorders.

18. The system of Claim 14 wherein the first algorithm is capable of processing input data comprising diagnostic wellness data obtained from a biofluid or tissue sample from the canine.

19. The system of Claim 14 further comprising:
(e) a fourth data set relating to contents of bioactive dietary components in food ingredients and, optionally, costs of the ingredients; and
(f) a second algorithm capable of selecting food ingredients from the fourth data set to define a food composition having a nutritional formula as derived by the first algorithm.

20. The system of Claim 19 wherein the second algorithm takes account of costs of ingredients to define a food composition having advantageous overall cost.

21. The system of Claim 19 further comprising (i) a computer-controlled mixing system capable of preparing the food composition defined by the second algorithm; and optionally (ii) a packaging system capable of placing a metered amount of the food composition in a suitable container, and (iii) a labeling system capable of printing a label or package insert with output data defining the breed cluster and optionally other attributes of the canine for which the food composition has been prepared and providing information on the nutritional formula and/or ingredients of the food composition.

## Patentansprüche

1. Verfahren zum Formulieren und Herstellen einer Nahrungszusammensetzung für einen Hund, umfassend:
(a) Identifizieren eines genombasierten Rasseclusters, zu dem der Hund gehört;
(b) Formulieren einer Nahrungszusammensetzung für den Hund mit einer Ernährungsformel, die zumindest zum Teil mit den Ernährungsbedürfnissen von Hunden in dem Rassecluster abgeglichen ist; und
(c) Herstellen einer Nahrungszusammensetzung für den Hund;
wobei der genombasierte Rassecluster aus der Gruppe bestehend aus Cluster I, Cluster II, Cluster III und Cluster IV wie in Parker et al., Science 304: 1160-1164, 2004 definiert ausgewählt ist,
und wobei Schritte (a) und (b) unter Verwendung eines computergestützten Systems durchgeführt werden.

2. Verfahren nach Anspruch 1, wobei die Ernährungsbedürfnisse zumindest zum Teil auf einem oder mehreren phänotypischen Merkmalen basieren, die für den Rassecluster charakteristisch sind, die aus der Gruppe bestehend aus Größe, Felltyp, Trainierbarkeit, Aktivitätslevel und Prävalenz von und Prädisposition für Krankheiten ausgewählt sind.

3. Verfahren nach Anspruch 1, das weiterhin Identifizieren von einem oder mehreren spezifischen zoografischen Merkmalen des Hundes umfasst, die aus der Gruppe bestehend aus Alter, Geschlecht, Größe, Gewicht, Felltyp, Stammbaum, Reproduktionsgeschichte, tiermedizinischer Geschichte, Appetit, umweltbezogenen Merkmalen und offensichtlichen vererblichen Zuständen und Störungen besteht; wobei die ausgewählte Nahrung eine Ernährungsformel aufweist, die modifiziert ist, um das/die spezifische(n) zoografische(n) Merkmal(e) zu berücksichtigen.

4. Verfahren nach Anspruch 3, wobei der Rassecluster und das/die spezifische(n) zoografische(n) Merkmal(e) des Hundes aus Eingabedaten identifiziert werden, die von einem Besitzer des Hundes bereitgestellt werden.

5. Verfahren nach Anspruch 4, wobei die Eingabedaten durch den Besitzer über eine Benutzeroberfläche eingegeben werden, die einen Computer, ein Bildschirmgerät mit Berührungseingabe, ein Telefon mit Berührungseingabe und/oder ein sprachgesteuertes System umfasst.

6. Verfahren nach Anspruch 1, das des Weiteren Identifizieren von einem oder mehreren spezifischen Wohlbefindensmerkmalen des Hundes umfasst, die aus der Gruppe bestehend aus Krankheitszuständen, Zuständen von Parasitenbefall, Haar- und Hautzustand, Sinnesschärfe, Veranlagungs- und Verhaltensmerkmalen und kognitiver Funktion ausgewählt sind; wobei die ausgewählte Nahrung eine Ernährungsformel aufweist, die modifiziert ist, um das/die spezifische(n) Wohlbefindensmerkmal(e) zu berücksichtigen.

7. Verfahren nach Anspruch 6, wobei das/die spezifische(n) Wohlbefindensmerkmal(e) des Hundes aus Eingabedaten identifiziert ist/sind, die durch einen Besitzer des Hundes und/oder einen tiermedizinischen Fachmann bereitgestellt sind.

8. Verfahren nach Anspruch 7, wobei die Eingabedaten diagnostische Daten umfassen, die aus einer biologischen Flüssigkeit oder einer Gewebeprobe des Hundes erhalten sind.

9. Verfahren nach Anspruch 1, das des Weiteren Mischen von Zutaten, die biologisch wirksame Ernährungsbestandteile bereitstellen, in mit der Ernährungsformel übereinstimmenden Mengen und Verhältnissen umfasst, um die Nahrung herzustellen.

10. Verfahren nach Anspruch 9, wobei die Nahrung ein Ergänzungsmittel darstellt, das zum Füttern in Verbindung oder im Gemisch mit einer Basisnahrung angepasst ist.

11. Verfahren nach Anspruch 9, wobei die Nahrung an einem Herstellungsort; an einem Verkaufsort; oder an einem Vertriebsort hergestellt wird und an einen Besitzer des Hundes geliefert wird.

12. Verfahren nach Anspruch 10, wobei die Nahrung durch einen Händler bereitgestellt wird oder durch einen Mischbetrieb bei Erhalt einer Verschreibung eines Tierarztes oder Ernährungsberaters, die die Ernährungsformel festlegt, hergestellt wird.

13. Verfahren nach Anspruch 12, wobei die Verschreibung einen Gutschein umfasst, der Gültigkeit zur Verwendung beim zumindest teilweisen Bezahlen der Nahrung besitzt, oder der den Inhaber des Gutscheins zu einer Ermäßigung oder einem Rabatt beim Kauf der Nahrung berechtigt.

14. Computergestütztes System, das ein bis zu einer Vielzahl an benutzeroberflächenfähigen Medien zum I mplementieren des Verfahrens gemäß einem beliebigen voranstehenden Anspruch umfasst, umfassend:
(a) einen ersten Datensatz, der eine Vielzahl an Rasseclustern mit genombezogenen Merkmalen jedes Rasseclusters in Beziehung setzt, wobei die genombasierten Rassecluster Cluster I, Cluster II, Cluster III und Cluster IV wie in Parker et al., Science 304: 1160-1164, 2004 definiert sind; und
(b) einen ersten Algorithmus, der unter Heranziehung des ersten Datensatzes fähig ist, (i) Eingabedaten zu einem oder mehreren genombezogenen Merkmalen des Hundes zu verarbeiten, um einen Rassecluster zu definieren, dem der Hund zugeordnet werden kann, und (ii) Formulieren einer Nahrungszusammensetzung für den Hund mit einer Ernährungsformel, die zumindest zum Teil mit den Ernährungsbedürfnissen von Hunden in dem Rassecluster abgeglichen ist.

15. System nach Anspruch 14, wobei die Eingabedaten einen oder mehrere genetische Marker umfassen, der/die individuell oder kollektiv einen Rassecluster anzeigt/anzeigen, wobei der eine oder die mehreren genetischen Marker durch Analyse einer biologischen Flüssigkeit oder einer Gewebeprobe, die aus dem Hund erhalten ist, abgeleitet ist/sind.

16. System nach Anspruch 14, das des Weiteren umfasst:
(c) einen zweiten Datensatz, der phänotypische Merkmale erfasst, die für jeden Rassecluster charakteristisch sind; und
(d) einen dritten Datensatz, der sich auf Wirkungen von biologisch aktiven Nahrungsbestandteilen (i) auf die phänotypischen Merkmale, wie sie durch spezifische zoografische Merkmale modifiziert werden, und gegebenenfalls (ii) auf spezifische Wohlbefindensmerkmale individueller Hunde bezieht;
wobei der erste Algorithmus des Weiteren unter Heranziehung des zweiten und dritten Datensatzes fähig ist, Eingabedaten von einem oder mehreren zoografischen Merkmalen und gegebenenfalls einem oder mehreren Wohlbefindensmerkmalen des Hundes zu verarbeiten, um eine Nahrungszusammensetzung für den Hund mit einer Ernährungsformel zu formulieren, die zumindest zum Teil mit Ernährungsbedürfnissen von Hunden in dem Rassecluster abgeglichen ist, und gegebenenfalls
wobei die Nahrungszusammensetzung zum Fördern von Wohlbefinden des Hundes geeignet ist.

17. System nach Anspruch 14, wobei die zoografischen Merkmale, die als Modifikatoren im dritten Datensatz wirken, ein oder mehrere Merkmale umfassen, die aus der Gruppe bestehend aus Alter, Geschlecht, Größe, Gewicht, Felltyp, Stammbaum, Reproduktionsgeschichte, tiermedizinischer Geschichte, Appetit, umweltbezogenen Merkmalen und offensichtlichen vererblichen Zuständen und Störungen ausgewählt sind.

18. System nach Anspruch 14, wobei der erste Algorithmus fähig ist, Eingabedaten zu verarbeiten, die diagnostische Wohlbefindensdaten umfassen, die aus einer biologischen Flüssigkeit oder einer Gewebeprobe des Hundes erhalten sind.

19. System nach Anspruch 14, das des Weiteren umfasst:
(e) einen vierten Datensatz, der sich auf Inhalte von biologisch wirksamen Ernährungsbestandteilen in Nahrungszutaten und gegebenenfalls auf die Kosten der Zutaten bezieht; und
(f) einen zweiten Algorithmus, der zum Auswählen von Nahrungsbestandteilen aus dem vierten Datensatz fähig ist, um eine Nahrungszusammensetzung mit einer wie durch den ersten Algorithmus abgeleiteten Ernährungsformel zu definieren.

20. System nach Anspruch 19, wobei der zweite Algorithmus Kosten von Zutaten berücksichtigt, um eine Nahrungszusammensetzung zu definieren, die vorteilhafte Gesamtkosten aufweist.

21. System nach Anspruch 19, umfassend des Weiteren (i) ein computergesteuertes Mischsystem, das zum Herstellen der durch den zweiten Algorithmus definierten Nahrungszusammensetzung fähig ist; und gegebenenfalls (ii) ein Verpackungssystem, das zum Platzieren einer abgemessenen Menge der Nahrungszusammensetzung in einen geeigneten Behälter fähig ist, und (iii) ein Kennzeichnungssystem, das zum Ausdrucken eines Etiketts oder einer Packungsbeilage mit Ausgabedaten fähig ist, die den Rassecluster und gegebenenfalls andere Merkmale des Hundes definieren, für den die Nahrungszusammensetzung hergestellt wurde, und die Information über die Ernährungsformel und/oder die Bestandteile der Nahrungszusammensetzung bereitstellen.

## Revendications

1. Procédé de formulation et de préparation d'une composition alimentaire pour un animal canin comprenant :
(a) l'identification d'un groupe de races basé sur le génome auquel appartient l'animal canin ;
(b) la formulation d'une composition alimentaire pour l'animal canin ayant une formule nutritionnelle correspondant au moins en partie aux besoins nutritionnels des animaux canins du groupe de races ; et
(c) la préparation d'une composition alimentaire pour l'animal canin ;
dans lequel le groupe de races basé sur le génome est choisi dans le groupe constitué par le groupe I, le groupe II, le groupe III et le groupe IV tels que définis dans Parker et al., Science 304 : 1160-1164, 2004,
et dans lequel les étapes (a) et (b) sont effectuées en utilisant un système assisté par ordinateur.

2. Procédé selon la revendication 1, dans lequel les besoins nutritionnels sont basés au moins en partie sur un ou plusieurs attributs phénotypiques caractéristiques du groupe de races choisis dans le groupe constitué par la taille, le type de pelage, l'aptitude au dressage, le niveau d'activité et la prévalence de et la prédisposition à des maladies.

3. Procédé selon la revendication 1, comprenant en outre l'identification d'un ou de plusieurs attributs zoographiques spécifiques de l'animal canin, choisis dans le groupe constitué par l'âge, le sexe, la taille, le poids, le type de pelage, le pedigree, les antécédents de reproduction, les antécédents médicaux vétérinaires, l'appétit, les attributs liés à l'environnement, et les conditions et les troubles héréditaires évidents ; dans lequel l'aliment choisi a une formule nutritionnelle modifiée pour tenir compte du ou des attributs zoographiques spécifiques.

4. Procédé selon la revendication 3, dans lequel le groupe de races et le ou les attributs zoographiques spécifiques de l'animal canin sont identifiés à partir de données d'entrée fournies par un propriétaire de l'animal canin.

5. Procédé selon la revendication 4, dans lequel les données d'entrée sont entrées par le propriétaire par l'intermédiaire d'une interface utilisateur comprenant un ordinateur, un terminal vidéo à écran tactile, un téléphone à touches et/ou un système à commande vocale.

6. Procédé selon la revendication 1, comprenant en outre l'identification d'un ou de plusieurs attributs de bien-être spécifiques de l'animal canin choisis dans le groupe constitué par les états pathologiques, les états d'infestation parasitaire, une affection du pelage et de la peau, l'acuité sensorielle, les attributs de disposition et de comportement, et la fonction cognitive ; dans lequel l'aliment choisi a une formule nutritionnelle modifiée pour tenir compte du ou des attributs de bien-être spécifiques.

7. Procédé selon la revendication 6, dans lequel le ou les attributs de bien-être spécifiques de l'animal canin sont identifiés à partir de données d'entrée fournies par un propriétaire de l'animal canin et/ou un professionnel vétérinaire.

8. Procédé selon la revendication 7, dans lequel les données d'entrée comprennent des données de diagnostic obtenues à partir d'un biofluide ou d'un échantillon de tissu provenant de l'animal canin.

9. Procédé selon la revendication 1, comprenant en outre l'incorporation d'ingrédients qui fournissent des constituants diététiques bioactifs en des quantités et dans des rapports compatibles avec la formule nutritionnelle, pour préparer l'aliment.

10. Procédé selon la revendication 9, dans lequel l'aliment constitue un complément adapté pour l'alimentation en conjonction ou en mélange avec un aliment de base.

11. Procédé selon la revendication 9, dans lequel l'aliment est préparé sur un site de fabrication ; sur un point de vente ; ou sur un site de distribution et remis à un propriétaire de l'animal canin.

12. Procédé selon la revendication 10, dans lequel l'aliment est fourni par un détaillant ou préparé par un préparateur à réception d'une prescription provenant d'un médecin vétérinaire ou d'un diététicien énonçant la formule nutritionnelle.

13. Procédé selon la revendication 12, dans lequel la prescription comprend un coupon validé pour être utilisé en paiement au moins en partie pour l'aliment, ou permettant à un porteur du coupon de bénéficier d'une réduction ou d'un rabais à l'achat de l'aliment.

14. Système assisté par ordinateur comprenant d'un support à une pluralité de supports pouvant être interfacés avec un utilisateur pour la mise en oeuvre du procédé selon l'une quelconque des revendications précédentes, comprenant :
(a) un premier ensemble de données reliant une pluralité de groupes de races à des attributs liés au génome de chaque groupe de races, dans lequel les groupes de races basés sur le génome sont le groupe I, le groupe II, le groupe III et le groupe IV tels que définis dans Parker et al., Science 304 : 1160-1164, 2004 ; et
(b) un premier algorithme apte, tout en se basant sur le premier ensemble de données, (i) à traiter des données d'entrée sur un ou plusieurs attributs liés au génome de l'animal canin pour définir un groupe de races auquel l'animal canin peut être attribué, et (ii) à formuler une composition alimentaire pour l'animal canin ayant une formule nutritionnelle correspondant au moins en partie aux besoins nutritionnels des animaux canins du groupe de races.

15. Système selon la revendication 14, dans lequel les données d'entrée comprennent un ou plusieurs marqueurs génétiques indiquant individuellement ou collectivement un groupe de races, le ou les marqueurs génétiques étant dérivés par analyse d'un échantillon de biofluide ou de tissu obtenu à partir de l'animal canin.

16. Système selon la revendication 14, comprenant en outre :
(c) un deuxième ensemble de données enregistrant des attributs phénotypiques caractéristiques de chaque groupe de races ; et
(d) un troisième ensemble de données concernant les effets des constituants diététiques bioactifs (i) sur les attributs phénotypiques tels que modifiés par des attributs zoographiques spécifiques, et éventuellement (ii) sur des attributs de bien-être spécifiques d'animaux canins individuels ;
dans lequel le premier algorithme est en outre apte, tout en se basant sur les deuxième et troisième ensembles de données, à traiter des données d'entrée sur un ou plusieurs attributs zoographiques et éventuellement un ou plusieurs attributs de bien-être de l'animal canin pour formuler une composition alimentaire pour l'animal canin ayant une formule nutritionnelle correspondant au moins en partie aux besoins nutritionnels des animaux canins du groupe de races, et éventuellement,
dans lequel la composition alimentaire est appropriée pour favoriser le bien-être de l'animal canin.

17. Système selon la revendication 14, dans lequel les attributs zoographiques agissant comme modificateurs dans le troisième ensemble de données comprennent un ou plusieurs attributs choisis dans le groupe constitué par l'âge, le sexe, la taille, le poids, le type de pelage, le pedigree, les antécédents de reproduction, les antécédents médicaux vétérinaires, l'appétit, les attributs liés à l'environnement, et les conditions et les troubles héréditaires évidents.

18. Système selon la revendication 14, dans lequel le premier algorithme est apte à traiter des données d'entrée comprenant des données de diagnostic de bien-être obtenues à partir d'un échantillon de biofluide ou de tissu provenant de l'animal canin.

19. Système selon la revendication 14, comprenant en outre :
(e) un quatrième ensemble de données concernant les teneurs en constituants diététiques bioactifs dans les ingrédients alimentaires et, éventuellement, les coûts des ingrédients ; et
(f) un deuxième algorithme apte à sélectionner des ingrédients alimentaires à partir du quatrième ensemble de données pour définir une composition alimentaire ayant une formule nutritionnelle telle que dérivée par le premier algorithme.

20. Système selon la revendication 19, dans lequel le deuxième algorithme tient compte des coûts des ingrédients pour définir une composition alimentaire ayant un coût global avantageux.

21. Système selon la revendication 19, comprenant en outre (i) un système de mélange commandé par ordinateur apte à préparer la composition alimentaire définie par le deuxième algorithme ; et éventuellement (ii) un système d'emballage apte à placer une quantité dosée de la composition alimentaire dans un récipient approprié, et (iii) un système d'étiquetage apte à imprimer une étiquette ou une notice avec des données de sortie définissant le groupe de races et éventuellement d'autres attributs de l'animal canin pour lequel la composition alimentaire a été préparée et fournissant des informations sur la formule nutritionnelle et/ou les ingrédients de la composition alimentaire.
